# EUROPEAN PATENT APPLICATION

(11) **EP 3 466 402 A1**
(43) Date of publication of application: **10.04.2019**
(21) Application number: 17806733.6
(22) Date of filing: 31.05.2017
(51) Int. Cl.: A61K 8/55, A61K 8/02, A61K 8/31, A61K 8/34, A61K 8/37, A61K 8/41, A61K 8/44, A61K 9/06, A61K 47/06, A61K 47/10, A61K 47/18, A61K 47/24, A61Q 1/04, A61Q 1/14, A61Q 5/12, A61Q 19/00, A61Q 19/10

(54) **OILY GELATINOUS COMPOSITION AND COSMETIC OR TOPICAL AGENT CONTAINING SAME**

(30) Priority: 03.06.2016 JP 2016111796
(71) Applicant: Nikko Chemicals Co., Ltd., Chuo-ku, Tokyo 103-0002 (JP); Nippon Surfactant Industries Co., Ltd., Chuo-ku, Tokyo 103-0002 (JP); Cosmos Technical Center Co., Ltd., Itabashi-ku, Tokyo 174-0046 (JP)
(72) Inventor: TANAKA Keisuke, Tokyo 174-0046 (JP)
(74) Representative: Gille Hrabal
(86) International application number: PCT/JP2017/020270
(87) International publication number: WO 2017/209182

(57) **Abstract**

To provide an oil gel composition wherein the viscosity of the oily ingredient is adjustable regardless of the type of oily ingredient, and also to provide a cosmetic or an external skin preparation containing this oil gel composition.

The oil gel composition containing the following ingredients (a) to (d) as essential ingredients:
(a) a monoalkyl phosphate;
(b) one or more neutralizing agents selected from basic amino acids and organic bases;
(c) water and/or a polyol; and
(d) an oily ingredient.

## Description

### Field of the Invention

The present invention relates to an oil gel composition and a cosmetic or an external skin preparation containing the same, using a self-assembling structure formed by a monoalkyl phosphate and a specific neutralizing agent, and a method for preparing the oil gel composition.

### Background of the Invention

Since oily ingredients generally have very different melting points, polarities, compatibilities, textures and utilities depending on their chemical structure and composition, the purposes of use of the respective oily ingredients differ.

In the cosmetic field, many oily ingredients, such as hydrocarbon oils, vegetable oils, animal oils, ester oils, silicone oils and essential oils, are used, and cosmetics and external preparations in which a large amount of an oily ingredient is blended to make the best use of its characteristics, such as texture and efficacy, are sold.

On the other hand, as mentioned above, since chemical structures and compositions are very different among oily ingredients, it is difficult to thicken the oily ingredients evenly and stably, and thus the development of materials capable of thickening evenly and stably oily ingredients having a wide variety of properties is desired.

As methods to thicken oily ingredients, mainly used are two methods: a method in which a thickener for oily ingredients is used and a method in which solidification is achieved by coexistence of a solid oil. Conventionally, dextrin fatty acid esters and inulin fatty acid esters are widely used as thickeners for oily ingredients (JP-A 4-49249 and JP-A 2005-145851).

However, these thickeners have the following disadvantages: their capability of thickening oily ingredients depends on the type and the polarity of oily ingredient to be thickened; there are variations in viscosity; long term stability cannot be maintained; the transparency after dissolution is low; they have to be blended in large amounts for sufficient thickening, which impacts the use feeling; they cannot thicken when using silicone oils or fluorine oils as oils; and the like.

The method in which solidification is achieved by coexistence of a solid oil also has the following disadvantages: the polarity of the solid oil and the liquid oil must be controlled; only solid formulations such as lipsticks and balms can be prepared; the transparency of the product is low, resulting in a white solid.

Meanwhile, monoalkyl phosphates are reported to easily form self-assembling structures such as α-gels and lamella liquid crystals in solvents including water and a polyol by using a basic amino acid or an organic salt as a neutralizing agent; however, it is not a technique to gelate oily ingredients, and oily ingredients are not blended in large amounts in cosmetics and external skin preparations (JP-A 2013-177367 and JP-A 2015-127322). Self-assembling structures formed by surfactants have also been applied to special emulsifying techniques such as liquid crystal emulsification and D-phase emulsification.

### Summary of the Invention

The present invention aims to prepare an oil gel composition in which a large amount of an oily ingredient is blended, to provide a gel composition stable regardless of the type of oily ingredient used, and to obtain a cosmetic or an external skin preparation containing this oil gel composition.

Considering these circumstances and as a result of intensive research to obtain an oil gel composition stable even when a large amount of an oily ingredient is blended, the inventor succeeded in preparing an oil gel composition stable regardless of the type of an oily ingredient and even when a large amount of an oily ingredient is blended, by combining (a) a monoalkyl phosphate, (b) one or more neutralizing agents selected from basic amino acids and organic bases, (c) water and/or a polyol, and (d) an oily ingredient, and also in obtaining a cosmetic or an external skin preparation containing this oil gel composition.

Namely, the present invention is an oil gel composition of a new product stable regardless of the type of the oily ingredient and even when a large amount of an oily ingredient is blended, by using a self-assembling structure formed by a monoalkyl phosphate and a neutralizing agent, and is also a cosmetic or an external skin preparation containing this oil gel composition.

The present invention is a novel oil gel composition that can thicken stably regardless of the type of an oily ingredient, which could not conventionally be achieved with an oil gel composition thickened by blending a large amount of a thickener for the oily ingredient, and further, this oil gel composition can adjust the viscosity over a wide range from low viscosity to high viscosity, which could not be obtained with the method in which a solid oil coexists.

Namely, an oil gel composition that is stable and makes the best use of the characteristics of an oily ingredient could be obtained, without using a thickener for an oily ingredient or a solid oil as an oil gelling agent, and various cosmetics and external skin preparations making the best use of the properties of the gel could be obtained by containing the oil gel composition.

### Embodiments of the Invention

The present invention is described below in detail.

Ingredient (a) used in the present invention is a monoalkyl phosphate, in which the alkyl group has preferably 12 to 32 carbon atoms, and more preferably 12 to 22 carbon atoms. It is easy to synthesize an alkyl phosphate in which the alkyl chain length is changed, and alkyl phosphates in which the alkyl chain length is arbitrarily changed can be synthesized. As ingredient (a), alkyl phosphates having different alkyl chain lengths can be used singly or in combinations of two or more.

Moreover, regardless of the alkyl group structure, any alkyl group, linear or branched, can be used. The alkyl phosphate of ingredient (a) can be synthesized using NIKKOL Phosten HLP (lauryl phosphate) or NIKKOL Purephos α (cetyl phosphate) from Nikko Chemicals Co., Ltd. as commercial products.

Ingredient (b) used in the present invention is one or more neutralizing agents selected from basic amino acids and organic bases. Specifically, examples of the basic amino acids include arginine, lysine, histidine and tryptophan, and one or more of these can be used. Examples of the organic bases include monoethanolamine, diethanolamine, triethanolamine, aminomethyl propanol, aminomethyl propanediol, aminoethyl propanediol and tris(hydroxymethyl)aminomethane, and one or more of these can be used. Among these, ingredient (b) is preferably one or more selected from arginine, triethanolamine and aminomethyl propanol, and especially preferably arginine.

It is preferable that ingredient (b) the one or more neutralizing agents selected from basic amino acids and organic bases be used in the range of 0.5 mol equivalent to 1.5 mol equivalent with respect to ingredient (a) the monoalkyl phosphate.

Moreover, the effect of the present invention is exhibited even if ingredient (a) the alkyl phosphate is already neutralized by ingredient (b) the one or more neutralizing agents selected from basic amino acids and organic bases. An example of a commercial product of such neutralized compound include NIKKOL Purephos LC (arginine hexyldecyl phosphate) from Nikko Chemicals Co., Ltd.

Ingredient (c) used in the present invention is a solvent including water and/or a polyol. Namely, in the present invention, one or more solvents selected from water and polyols can be used as ingredient (c). Examples of the polyol include glycerin, diglycerin, 1,3-butylene glycol, propylene glycol, dipropylene glycol, sorbitol, xylitol, maltitol and polyethylene glycol, and one or a combination of two or more of these can be used. Among these, ingredient (c) is preferably one or more selected from water, glycerin, butylene glycol and sorbitol.

As ingredient (d) the oily ingredient used in the present invention, hydrocarbon oils such as squalane, liquid paraffin and isoparaffin; ester oils such as glyceryl triisooctanoate, isopropyl myristate, cetyl isooctanoate and isooctyl palmitate; vegetable oils such as olive oil, macadamia nut oil and jojoba oil; silicone oils such as dimethyl silicone, phenylmethyl silicone, cyclomethicone and amodimethicone, and fluorine oils, which are liquid oils, can be used singly or in combinations of two or more, regardless of the type of oil. Semisolid oils such as vaseline and shea butter can also be blended by dissolving them in a liquid oil.

The oil gel composition in the present invention preferably contains 35 to 98% by mass, more preferably 50 to 98% by mass of ingredient (d) in the formulation.

The oil gel composition of the present invention can be prepared, for example, by mixing evenly ingredient (a) and ingredient (b) in ingredient (c), then gradually adding ingredient (d) while stirring ingredients (a) to (c) .

Namely, the present invention provides a method for preparing an oil gel composition characterized in that the composition is obtained by adding ingredient (d) the oily ingredient to the mixture of ingredient (a), ingredient (b) and ingredient (c), while stirring.

The oil gel composition of the present invention has a viscosity measured by a B-type viscometer at room temperature (25°C) after preparation of preferably 3000 mPa·s or more, and more preferably 5000 mPa·s or more. The viscosity can be adjusted arbitrarily by the amount of the oil blended or the composition of the other ingredients blended.

The oil gel composition of the present invention is preferably contained in a formulation that is a cosmetic or an external skin preparation. Namely, the present invention is a cosmetic or an external skin preparation containing the oil gel composition of the present invention. Moreover, the oil gel composition of the present invention can be used as a cosmetic or an external skin preparation.

The cosmetic or external skin preparation of the present invention can be prepared by preparing beforehand the oil gel composition of the present invention and adding other ingredients mentioned below to the composition.

In the cosmetic or external skin preparation containing the oil gel composition of the present invention, various ingredients used conventionally in cosmetics and external skin preparations, such as, for example, nonionic surfactants, polar lipids, active ingredients, moisturizing ingredients, antibacterial ingredients, viscosity control agents, synthetic pigments, colored pigments, pearlescent agents and perfume can be blended.

Examples of the nonionic surfactants include POE alkyl ethers, POE fatty acid esters, POE sorbitol fatty acid esters, POE glycerol fatty acid esters, POE/POP alkyl ethers, polysorbates, sorbitan fatty acid esters, polyglycerol fatty acid ester, POE-modified silicone, sucrose fatty acid esters and glycerol fatty acid esters.

Examples of the polar lipids include ceramides, phospholipids, cholesterol and derivatives thereof, and glycolipids.

Examples of the active ingredients include whitening agents such as ascorbic acid, ascorbic phosphoric ester magnesium, ascorbyl palmitate, ascorbyl stearate, ascorbyl tetraisopalmitate, ascorbic acid glucoside, arbutin, ellagic acid or rucinol; NMF ingredients such as amino acids; rough skin inhibitors such as water-soluble collagen, elastin, glycyrrhizinic acid, glycyrrhetinic acid or ceramides; anti-aging agents such as retinol or vitamin A acid, and various vitamins and their derivatives.

Examples of the moisturizing ingredients include chondroitin sulfate, hyaluronic acid and sodium pyrrolidone carboxylate.

Examples of the synthetic pigments include Red 104, 201, 202, 204, 213, 220, 226 and 227, Yellow 4 and Blue 1.

Examples of the colored pigments include red iron oxide, yellow iron oxide, black iron oxide, titanium oxide, ultramarine blue and Prussian blue.

Examples of the pearlescent agents include polarizing pearlescent agents consisting of mica or titanium oxide, colored pearlescent agents consisting of iron oxide, titanium oxide and mica, glass powder and laminate.

Moreover, examples of the cosmetics or external skin preparation containing the oil gel composition of the present invention include skin care cosmetics such as beauty oils, essences, milks, creams, cleansers, massage agents and sunscreen agents, hair care cosmetics such as treatments, conditioners and hair dressing agents, lip care products such as lipsticks and lip glosses, but are not limited thereto.

### Examples

The present invention is further explained below in detail while showing Examples, but the present invention is not limited thereto. Moreover, % by mass shown below refers to % by mass relative to the whole composition.

### <Example 1>

Oil gel compositions were prepared with the compositions (% by mass) shown in Table 1 and 2. The oil gel compositions were prepared by evenly mixing Part (A), Part (B) and Part (C), then adding gradually Part (D), while continuing stirring.

The prepared oil gel compositions were evaluated as follows.

### (1) Oil gel formation

After preparation, the appearance was checked and was evaluated as good when it had incorporated all the oils, and as poor when there was a crystal deposition or when all the oils were not incorporated.

### (2) Viscosity measurement

Each composition was charged in a wide mouth bottle and the viscosity was measured using a B-type viscometer at 25°C.

### (3) Stability evaluation

The appearance of the formulation and the change in viscosity were evaluated after storing under conditions of room temperature, 5°C and 45°C, each for 1 month. Those that were stable with no change in the appearance and the viscosity after 1 month were evaluated as good, and those that were unstable, with a significant change in the appearance and the viscosity were evaluated as poor.

**[Table 1]**

| Formulation ingredient | | Example | | | | |
|---|---|---|---|---|---|---|
| | | 1-1 | 1-2 | 1-3 | 1-4 | 1-5 |
| (A) | Lauryl phosphate | 0.80 | | | 0.30 | |
| | Cetyl phosphate | 0.40 | | | | |
| | Behenyl phosphate | | | | 2.00 | |
| | Hexyldecyl phosphate | | 2.00 | 1.20 | | 1.40 |
| | Octyldodecyl phosphate | | | 0.20 | | |
| (B) | Arginine | 0.50 | | 0.30 | 1.00 | 0.70 |
| | Triethanolamine | | 1.00 | | | |
| | Aminomethyl propanol | | | 0.30 | | |
| (C) | Purified water | 5.00 | | 15.00 | 10.00 | 20.00 |
| | Glycerin | 8.00 | 10.00 | | 3.00 | 35.00 |
| | Butylene glycol | 1.00 | | 3.00 | | |
| | Sorbitol | | | 0.50 | | |
| (D) | Squalane | Balance | | | Balance | |
| | Isooctyl palmitate | 35.00 | Balance | | | 15.00 |
| | Macadamia nut oil | | | Balance | | |
| | Dimethicone (10cs) | | 25.00 | | | Balance |
| | Cyclopentasiloxane | | 25.00 | | | |
| | White Vaseline | 1.50 | | | | |
| | Shea butter | | | 3.00 | | |
| Total | | 100.00 | 100.00 | 100.00 | 100.00 | 100.00 |
| Oil gel formation | | Good | Good | Good | Good | Good |
| Viscosity (mPa · s) | | 95000 | 88000 | 58000 | 90000 | 5000 |
| Stability test | | Good | Good | Good | Good | Good |

**[Table 2]**

| Formulation ingredient | | Comparative Example | | | | |
|---|---|---|---|---|---|---|
| | | 1-1 | 1-2 | 1-3 | 1-4 | 1-5 |
| (A) | Lauryl phosphate | 1.30 | | | | |
| | Cetyl phosphate | 0.80 | | | 1.00 | |
| | Hexyldecyl phosphate | | | 2.00 | | |
| | Palmitic acid | | | | | 2.00 |
| (B) | Arginine | 0.90 | | | | |
| | Triethanolamine | | 3.00 | | | 1.00 |
| | Potassium hydroxide | | | | 0.70 | |
| (C) | Purified water | | 5.00 | 10.00 | | 7.00 |
| | Glycerin | | 12.00 | | 8.00 | 7.00 |
| | Butylene glycol | | | 3.00 | | |
| | Sorbitol | | | 3.00 | 5.00 | |
| (D) | Squalane | 40.00 | Balance | | | 30.00 |
| | Isooctyl palmitate | 20.00 | | | Balance | 30.00 |
| | Macadamia nut oil | | | Balance | 15.00 | |
| | Dimethicone (10cs) | | 30.00 | | | |
| | Cyclopentasiloxane | Balance | | | | Balance |
| | White Vaseline | | 2.00 | | | |
| | Shea butter | | | 3.50 | | |
| Total | | 100.00 | 100.00 | 100.00 | 100.00 | 100.00 |
| Oil gel formation | | Poor | Poor | Poor | Poor | Poor |
| Viscosity (mPa · s) | | - | - | - | - | - |
| Stability test | | - | - | - | - | - |

### <Example 2>

**Skin care spot gel**

| | |
|---|---|
| (A) Arginine hexyldecyl phosphate | 1.00 (% by mass) |
| Polyglyceryl-10 laurate | 1.00 |
| Squalane | 10.00 |
| Glycerin | 6.30 |
| Water | balance |
| Preservative | suitable amount |
| (B) Caprylic/capric triglyceride | 3.00 |
| Tocopheryl retinoate | 0.10 |
| Pyridoxine tris-hexyldecanoate | 1.00 |
| Squalane | 75.00 |
| Total | 100.00 |

Preparation method: Phase A was stirred and mixed evenly. Phase B was added gradually while stirring to form a gel, stirring was further continued after adding all phase B, and the preparation was completed when evenly mixed.

The following evaluations were performed with the same method as in Example 1.

Oil gel formation: An even oil gel formation was confirmed.

Viscosity (mPa·s) : 89000

Stability evaluation: Stable, with no decrease in viscosity, crystal deposition or separation of the oils observed at each temperature.

### <Example 3>

**Cleansing oil gel**

| | |
|---|---|
| (A) Arginine hexyldecyl phosphate | 1.50 (% by mass) |
| Polyglyceryl-10 laurate | 5.00 |
| Squalane | 10.00 |
| Glycerin | 15.00 |
| Water | 7.00 |
| Preservative | suitable amount |
| (B) Caprylic/capric triglyceride | balance |
| Isododecane | 15.00 |
| Total | 100.00 |

Preparation method: Phase A was stirred and mixed evenly. Phase B was gradually added while stirring to form a gel, stirring was further continued after adding all the phase B, and the preparation was completed when evenly mixed.

The following evaluations were performed with the same method as in Example 1.

Oil gel formation: An even oil gel formation was confirmed.

Viscosity (mPa·s) : 63400

Stability evaluation: Stable, with no decrease in viscosity, crystal deposition or separation of the oils observed at each temperature.

### <Example 4>

**Body massage gel**

| | |
|---|---|
| (A) Cetyl phosphate | 0.70 (% by mass) |
| Glycerin | 10.50 |
| Preservative | suitable amount |
| (B) Water | 4.50 |
| Arginine | 0.35 |
| (C) NIKKOL Aromasqualane Rose* | balance |
| Tocopherol | 0.50 |
| Mica-type pearlescent agent | 3.00 |
| Total | 100.00 |

| | |
|---|---|
| * Mixture of squalane and rosa damascena flower extract | |

Preparation method: Phase A and phase B were each dissolved under heating and stirred, then mixed evenly. After mixing phase A and phase B, phase C was gradually added while stirring to form a gel, stirring was further continued after adding all the phase C, and the preparation was completed when evenly mixed.

The following evaluations were performed with the same method as in Example 1.

Oil gel formation: An even oil gel formation was confirmed.

Viscosity (mPa·s): 100,000 or more

Stability evaluation: Stable, with no decrease in viscosity, crystal deposition or separation of the oils observed at each temperature.

### <Example 5>

**Out bath hair treatment**

| | |
|---|---|
| (A) Arginine octyldodecyl phosphate | 1.00 (% by mass) |
| Glycerin | 9.00 |
| Water | 4.00 |
| Preservative | suitable amount |
| (B) C13-15 Alkane | balance |
| High polymer dimethicone | 10.00 |
| Amodimethicone | 2.00 |
| Laureth-9 | 1.00 |
| Total | 100.00 |

Preparation method: Phase A was stirred and mixed evenly. Phase B was gradually added while stirring to form a gel, stirring was further continued after adding all the phase B, and the preparation was completed when evenly mixed.

The following evaluations were performed with the same method as in Example 1.

Oil gel formation: An even oil gel formation was confirmed.

Viscosity (mPa·s) : 98000

Stability evaluation: Stable, with no decrease in viscosity, crystal deposition or separation of the oils observed at each temperature.

### <Example 6>

**Out bath hair treatment (Low viscosity type)**

| | |
|---|---|
| (A) Hexyldecyl phosphate | 1.00 (% by mass) |
| Triethanolamine | 0.50 |
| Glycerin | 19.50 |
| Water | 8.50 |
| Preservative | suitable amount |
| (B) C13-15 Alkane | balance |
| High polymer dimethicone | 15.00 |
| Total | 100.00 |

Preparation method: Phase A was stirred and mixed evenly. Phase B was gradually added while stirring to form a gel, stirring was further continued after adding all the phase B, and the preparation was completed when evenly mixed.

The following evaluations were performed with the same method as in Example 1.

Oil gel formation: An even oil gel formation was confirmed.

Viscosity (mPa·s) : 12000

Stability evaluation: Stable, with no decrease in viscosity, crystal deposition or separation of the oils observed at each temperature.

### <Example 7>

**Lip gloss**

| | |
|---|---|
| (A) Cetyl phosphate | 1.50 (% by mass) |
| Glycerin | 10.00 |
| Preservative | suitable amount |
| (B) Water | 4.00 |
| Aminomethyl propanol | 0.70 |
| (C) Squalane | 25.00 |
| Diisostearyl malate | 25.00 |
| Hydrogenated polyisobutene | balance |
| Jojoba seed oil | 5.00 |
| Hazelnut oil | 1.00 |
| Pyridoxine tris-hexyldecanoate | 1.00 |
| Stearyl glycyrrhetinate | 0.10 |
| Synthetic pigment | suitable amount |
| (D) Polyperfluoromethylisopropyl ether | 5.00 |
| Total | 100.00 |

Preparation method: Phase A and phase B were each dissolved under heating and stirred, then mixed evenly. After mixing phase A and phase B, phase C and phase D were gradually added while stirring to form a gel, stirring was further continued after adding all the phase D, and the preparation was completed when evenly mixed.

Oil gel formation: An even oil gel formation was confirmed.

The following evaluations were performed with the same method as in Example 1.

Viscosity (mPa·s): 100,000 or more

Stability evaluation: Stable, with no decrease in viscosity, crystal deposition or separation of the oils observed at each temperature.

### <Example 8>

**Watery lip gel**

| | |
|---|---|
| (A) Cetyl phosphate | 1.50 (% by mass) |
| Cetyl Alcohol | 0.25 |
| Glycerin | 15.00 |
| Preservative | suitable amount |
| (B) Water | 12.00 |
| Arginine | 0.75 |
| (C) Squalane | 25.00 |
| Diisostearyl malate | 25.00 |
| Hydrogenated polyisobutene | balance |
| Jojoba seed oil | 5.00 |
| Hazelnut oil | 1.00 |
| Pyridoxine tris-hexyldecanoate | 1.00 |
| Stearyl glycyrrhetinate | 0.10 |
| Colored pigment | suitable amount |
| Synthetic pigment | suitable amount |
| Pearlescent agent | suitable amount |
| Total | 100.00 |

Preparation method: Phase A and phase B were each dissolved under heating and stirred, then mixed evenly. After mixing phase A and phase B, phase C was gradually added while maintaining in a heated state and stirring to form a gel, stirring was further continued after adding all the phase C, and the preparation was completed when evenly mixed.

The following evaluations were performed with the same method as in Example 1.

Oil gel formation: An even oil gel formation was confirmed.

Viscosity (mPa·s) : 83, 000

Stability evaluation: Stable, with no decrease in viscosity, crystal deposition or separation of the oils observed at each temperature.

### <Example 9>

**Nonaqueous oil gel pack**

| | |
|---|---|
| (A) Arginine hexyldecyl phosphate | 0.70 (% by mass) |
| Glycerin | 30.00 |
| Preservative | suitable amount |
| (B) Squalane | balance |
| Total | 100.00 |

Preparation method: Phase A was dissolved under heating and stirred, then mixed evenly. Phase B was gradually added while stirring to form a gel, stirring was further continued after adding all the phase B, and the preparation was completed when evenly mixed.

The following evaluations were performed with the same method as in Example 1.

Oil gel formation: An even oil gel formation was confirmed.

Viscosity (mPa·s): 100,000 or more

Stability evaluation: Stable, with no decrease in viscosity, crystal deposition or separation of the oils observed at each temperature.

### Industrial Applicability

The oil gel composition of the present invention can provide a wide range of cosmetics and external skin preparations from low viscosity to high viscosity, that are stable regardless of the type of oily ingredient.

## Claims

1. An oil gel composition **characterized by** comprising the following ingredients (a) to (d) as essential ingredients:
(a) a monoalkyl phosphate;
(b) one or more neutralizing agents selected from basic amino acids and organic bases;
(c) water and/or a polyol; and
(d) an oily ingredient.

2. The oil gel composition according to claim 1, wherein the alkyl group of the ingredient (a) monoalkyl phosphate has a linear or branched structure having 12 to 36 carbon atoms.

3. The oil gel composition according to claim 1 or 2, having a viscosity of 3000 mPa·s or more.

4. A cosmetic comprising the oil gel composition according to any one of claims 1 to 3.

5. An external skin preparation comprising the oil gel composition according to any one of claims 1 to 3.

6. A method for preparing an oil gel composition, **characterized in that** the composition is obtained by adding an ingredient (d) an oily ingredient to a mixture of an ingredient (a) a monoalkyl phosphate, an ingredient (b) one or more neutralizing agents selected from basic amino acids and organic bases, and an ingredient (c) water and/or a polyol, while stirring.
